# EUROPEAN PATENT APPLICATION

(11) **EP 2 993 475 A1**
(43) Date of publication of application: **09.03.2016**
(21) Application number: 14791963.3
(22) Date of filing: 30.04.2014
(51) Int. Cl.: G01N 33/68, C12Q 1/68

(54) **METHOD FOR OBTAINING USEFUL DATA FOR THE DIFFERENTIAL DIAGNOSIS OF LIVER FIBROSIS**

(30) Priority: 30.04.2013 ES 201330636
(71) Applicant: Fundación Pública Andaluza Progreso Y Salud, 41092 Sevilla (ES); Fundación Pública Andaluza Para la Gestión de la Investigación en Salud en Sevilla, 41013 Sevilla (ES); Universidad Pablo de Olavide, 41013 Sevilla (ES); Universidad de Sevilla, 41012 Sevilla (ES); Servicio Andaluz de Salud, 41071 Sevilla (ES)
(72) Inventor: ROJAS GONZÁLEZ, Ana Isabel, 41092 Sevilla (ES); CANO GONZÁLEZ, David, 41013 Sevilla (ES); DELGADO SAINZ, Irene, 41092 Sevilla (ES); SORIA ESCOMS, Bernat, 41092 Sevilla (ES); MARTÍN BERMUDO, Francisco, 41013 Sevilla (ES); ROMERO GÓMEZ, Manuel, 41013 Sevilla (ES)
(74) Representative: Hoffmann Eitle
(86) International application number: PCT/ES2014/070378
(87) International publication number: WO 2014/177747

(57) **Abstract**

The invention relates to a method for the diagnosis of liver fibrosis, comprising detecting the level of expression of the gene GATA-4, or the quantity of the protein GATA-4, in the isolated biological sample of liver tissue. The invention also relates to a diagnosis kit.

## Description

### TECHNICAL FIELD

The present invention is within the medicine and molecular biology field, and refers to a method for obtaining useful data for the early diagnosis of hepatic fibrosis, as well as to evaluate the response to treatment of said disease, enabling the establishment of a single pattern of (quantitative) specific recognition, which is post-treatment modified, allowing the establishment of groups of patients.

### BACKGROUND OF THE INVENTION

Chronic liver disease is currently a leading cause of hospitalization and mortality, especially in patients who are co-infected with HIV and HCV or HBV. The incidence of hepatic disease is increasing.

Hepatic fibrosis is a pathophysiological response to chronic damage caused mainly by the abusive consumption of alcohol, viral infection or obstruction of the bile duct. In the fibrotic process the accumulation of components of the extracellular matrix, such as collagen and laminin, occurs. The content in components of extracellular matrix is controlled by multiple molecular and cellular mechanisms, including the proliferation and activation of collagen producing cells. The largest source of collagen producing cells and other components of the extracellular matrix are the stellate cells of the liver (Hepatic Stellate Cells, HSCs). Under normal conditions, HSCs are in a quiescent state and store vitamin A. After liver damage, these cells respond by activating and acquiring a myofibroblast form, they enter into cell cycle and begin to secrete extracellular matrix components. The deposition of collagen fibres helps to form scars, which disrupt the liver structure and affects, therefore, the metabolic function of hepatocytes. Hepatic fibrosis can be reversed if the damage-causing agent disappears. In this case, a mechanism of liver regeneration is triggered, which includes the reversion to the inactive state of HSCs and replication of the hepatocytes that replace the loss of functional liver tissue. However, if the liver damage is maintained, the progression of hepatic fibrosis entails an irreversible disease stage, known as cirrhosis, in which the excessive accumulation of extracellular matrix components distorts the liver architecture and the liver is unable to respond to the metabolic demands of the organism.

Fibrosis, therefore, is a frequent phenomenon in liver diseases and although it is of non-specific nature, its exact characterisation is of help for the diagnosis. Its assessment includes defining its location, distribution and intensity. Its location can be portal, peri-portal, intra-acinar, zonal, sinusoidal, in bridge form, or peri-nodular. With respect to its distribution, it can be focal, diffuse, lobar, and segmental. The intensity is classified as mild, moderate, intense or severe, and cirrhotic.

An accurate diagnosis of the grade of fibrosis is necessary to determine the progression of liver disease, make a prognosis and take therapeutic decisions.

Currently, liver biopsy is the reference method for determining the degree of hepatic fibrosis and has been widely recommended to assess the need to treat patients with one or other treatment. However, non-invasive procedures have been developed to determine the different stages of hepatic fibrosis, which can be divided into two categories: imaging methods, such as the transient elastometry, and tests based on serum biomarkers definition.

Thus, the Fibrotest is an index that is calculated by combining 5 indirect biochemical fibrosis markers adjusted to age and sex. These markers are associated with activation of HSCs. HSCs are the main liver fibrogenic cells. Currently, the main therapeutic strategies to prevent or reverse the hepatic fibrosis are based either in inhibiting the activation or proliferation of HSCs, or in inducing apoptosis in the activated cells. The APRI test refers to the index of the relationship of the aspartate aminotransferase (AST) and platelets. It is based on the fact that the progressive fibrosis is associated with decrease in the elimination of AST and thrombocytopenia. FIB-4 is a simple index that includes age, levels of AST, levels of minotransferase (ALT), and platelet count. HGM1 and HGM2 are two simple indexes for the diagnosis of hepatic fibrosis, based on routine laboratory data. HGM-1 is based on platelet count, AST and serum glucose levels on fasting. HGM-2 is based on platelet count, INR index, alkaline phosphatase (ALP) and AST.

There are also a number of direct serum markers, such as zinc hyaluronate (zHA), collagen type IV, procollagen type III N-terminal propeptide provol (PIIINP), matrix metalloproteinases (MMPs), proteolytic enzymes secreted by the HSCs involved in the destruction of the extracellular matrix (ECM), tissue inhibitors of matrix metalloproteases (TIMPs), laminin, or human cartilage 39-glycoprotein (YKL-40).

The European Group of Liver Fibrosis (ELF) has developed an algorithm using the age, serum levels of zHA, PIIINP and TIMP-1. Another measurement model for hepatic fibrosis is Hepascore, based on the levels of 4 serum markers: bilirubin, gamma-glutamyl transpeptidase (GGT), zHA and alpha-2-macroglobulin, taking into account the age and sex.

Currently, hepatic fibrosis is also diagnosed by transient elastography (Fibroscan®). It consists of a new diagnostic technique of hepatic fibrosis using a probe that emits and receives ultrasonic waves. The speed of transmission of these ultrasounds through a specific medium varies with the density and elasticity of said medium. Using a physical equation, liver elasticity can be computerised based on the transmission speed, in such a way that the higher is the speed, lower the elasticity and greater the rigidity of the liver (fibrosis), the latter being an opposite quality to elasticity.

Liver biopsy is currently the diagnostic technique of choice to determine the presence and condition of hepatic fibrosis. There are several systems to quantify the stage of hepatic fibrosis, such as the histological analysis by staining with hematoxylin and eosin or Sirius red to dye the collagen fibres of the biopsy specimens. The most widely used is METAVIR (METAVIR scoring system). METAVIR measure fibrosis on a scale of 0-4, where F0 corresponds to absence of fibrosis; F1, portal fibrosis without septae; F2, portal fibrosis with a few septae; F3, numerous septae without cirrhosis; and F4, cirrhosis. A fibrosis is considered significant when the stage of fibrosis is equal to or greater than F2, and advanced fibrosis when reporting a fibrosis stage equal to or greater than F3. Detection of patients in stage F2 or greater involves important clinical implications because, in the majority of cases, it is considered the threshold for starting treatment. In addition to the histological analysis, the search for markers that confirm the presence of the fibrotic process becomes necessary.

The transcription factor GATA-4 or GATA binding protein 4 (also known as ASD2 and VSD1) is a member of the family of transcription factors of the zinc-finger type, which is expressed during embryonic development in various organs such as the heart, pancreas and liver, and its expression continues in adult stages. Members of this family recognize the GATA motif, which is present in the promoters of many genes. This protein is believed to regulate genes involved in embryogenesis and myocardium differentiation and function. Mutations in this gene have been associated with cardiac septal defects. This gene is located on human chromosome 8 (8p23.1-p22).

The detection of the presence of hepatic fibrosis and quantification of its magnitude are crucial for making decisions related to the clinical management of liver disease. Since individuals who have mild or moderate fibrosis have more likelihood of the treatment being more effective and of the disease stabilising and even reversing, it is necessary to find a marker that allows the early detection of patients with this type of fibrosis.

### DESCRIPTION OF THE INVENTION

Immunohistochemical analyses carried out in the laboratory of the inventors of the present invention have shown that GATA-4 is expressed in the stellate cells of the adult liver in humans in non-pathological conditions. However, the expression of GATA-4 decreases in fibrotic advanced stages. This decrease is even more dramatic in cirrhotic stages of biopsies of liver diseases by C virus, and especially in alcoholic liver diseases.

The authors of the present invention have developed a method and a kit that uses a marker that is sensitive enough to discriminate between the early and late stages of fibrosis and the cirrhosis stage.

The examples of the present invention show the Immunohistochemical localisation of the GATA-4 protein involved in the hepatic fibrosis, in tissue samples from patients.

This protein staining pattern was different among patients with different stages of evolution of hepatic fibrosis and cirrhosis.

Therefore, a first aspect of the invention relates to the use of the GATA-4 gene, or GATA-4 protein for the differential diagnosis of fibrosis and cirrhosis.

### METHOD FOR OBTAINING USEFUL DATA AND METHOD OF DIFFERENTIAL DIAGNOSIS

Another aspect of the invention relates to a method for obtaining useful data for the differential diagnosis of fibrosis and cirrhosis comprising:
a) obtaining an isolated biological sample comprising cells from an individual,
b) detecting the level of expression of the GATA-4 gene, and/or detecting the amount of GATA-4 protein, in the isolated sample of (a).
c) comparing the gene expression of step (b) with a reference amount.

The steps (b) and/or (c) of the methods described above may be completely or partially automated, for example, through a sensor robotic instrument for detecting the amount in step (b) or the computerised comparison in step (c).

Another aspect of the invention relates to a method for the differential diagnosis of hepatic fibrosis and cirrhosis in an individual, from now on second method of the invention, which comprises the steps (a) - (c) according to the first method of the invention, and further comprises:
d) diagnosing the individual of step (a) as an individual with portal fibrosis without septae (F1), when it presents an increased expression of the GATA-4 gene or a larger amount of GATA-4 protein in the sample obtained in (a), in relation to the amount of expression detected for this gene or protein in a population of patients of reference. In a preferred embodiment of this aspect of the invention, the expression of the GATA-4 gene, or a larger amount of GATA-4 protein is detected in the nucleus of the stellate cells. More preferably, the individual of step (a) presents an expression level of the GATA-4 gene of 110 % over the expression levels in a population of patients (FO) of reference.

In another preferred embodiment, the individual of step (a) is diagnosed as an individual with portal fibrosis with numerous septae without cirrhosis, portal fibrosis with a few septae (FII) or many septae (FIII), when it presents an expression level of the GATA-4 gene between 15 % and 25 % less than in an individual of reference. More preferably, it presents an expression level of the GATA-4 gene approximately 20 % less than the individual of reference (F0).

In another preferred embodiment of this aspect of the invention, the individual of step (a) is diagnosed as an individual with cirrhosis (FIV), when it presents an expression level of the GATA-4 gene between 55 % and 65 % less than an individual of reference. More preferably, the individual presents an expression level of the GATA-4 gene approximately 60 % less than the individual of reference.

An isolated "biological sample" includes, but without being limited to, cells, tissues and/or biological fluids of an organism, obtained by any method known to a person skilled in the art.

In a preferred embodiment of this aspect of the invention, the isolated biological sample from an individual of step (a) is a tissue sample, preferably liver tissue.

Detection of the expression of genes, or the detection of the amount of protein, can be carried out by any means known in the state of the art.

Measurement of the concentration, preferably in a quantitative way, can be carried out directly or indirectly. Direct measurement means the measurement of the expression of genes based on a signal that is obtained directly from the transcripts of these genes, or from proteins to which they are translated, and which is directly correlated with the number of molecules of RNA or proteins produced by the genes. This signal - which also can be referred to as intensity signal- can be obtained, for example, by measuring a value of intensity of a chemical or physical property of such products. The indirect measurement includes the measurement obtained from a secondary component or a system of biological measurement (for example measurement of cellular responses, ligands, 'tags', or enzymatic reaction products).

The term "comparison", as used in the description, refers, but is not limited to the comparison of the expression levels of the GATA-4 gene in the biological sample to be analysed, also called problem biological sample, with the expression levels of the GATA-4 gene of one or more specimens of desirable reference described elsewhere in the present description. The reference sample can be analysed, for example, simultaneously or consecutively, along with the problem biological sample. The comparison described in paragraph (c) of the method of the present invention, can be computer-assisted.

The expression levels of genes or proteins will give a particular profile of gene or protein expression. The term "expression level", refers to the biochemical material, either RNA or protein, resulting from the expression of a gene. Sometimes a measurement of the amount of gene product is used to infer how active a gene is. "Gene expression profile" means the gene profile obtained after the quantification of mRNA produced by the genes of interest or biomarkers, i.e., by the GATA-4 gene, in a biological sample. The gene expression profile is made, preferably, by determining the level of mRNA derived from its transcription, prior to extraction of total RNA in the isolated biological sample, which can be carried out by using protocols known in the state of the art. The determination of the level of mRNA from the transcription of the GATA-4 gene can be done, for example, but without being limited to, amplification by polymerase chain reaction (PCR), retrotranscription in combination with the polymerase chain reaction with retrotranscription (RT-PCR), direct mRNA sequencing, series analysis of gene expression (SAGE, SuperSAGE); RNA chips made using oligonucleotides deposited by any mechanism; RNA microarrays made using oligonucleotides synthesised in-situ by photolithography or by any other mechanism; hybridisation in situ using specific probes for the GATA-4 gene labelled with any labelling method; by electrophoresis gels; by transfer to membrane and hybridisation with a specific probe; by nuclear magnetic resonance or any other technique of diagnostic imaging using para-magnetic nanoparticles or any other type of detectable nanoparticles functionalised with antibodies, or by any other means. Protein expression profile refers to that obtained using the detection and/or quantification of the protein or proteins, which are product of the mRNA translation derived from the transcription of the GATA-4 genes, by means of, for example, but not limited to, western blot immunodetection. The quantitative detection of the expression of the GATA-4 gene can be done more preferably by PCR in real time (RT-PCR or RTqPCR). Detection in real time of the amplified products can be performed through the use of fluorescent molecules that are interspersed in the double-stranded DNA or through hybridisation with different types of probes.

More preferably, the detection of the expression levels of the GATA-4 gene is performed using Q-RT-PCR.

Quantitative PCR in real time (Q-RT-PCR) is a technique for quantification of sensitive and reproducible gene expression that can be used in particular for the expression of the gene profile in cells and tissues. Any procedure can be used for the evaluation of the results of the RT-PCR, and the ΔΔCt procedure may be preferred. The ΔΔCT procedure is described in detail in Livak and col. (Methods 2001, 25:402-408). (Ct = cycle threshold values). When the present invention is implemented, the ΔΔCT procedure should be used preferably as described by Livak and col. (Methods 2001, 25:402-408). The ΔΔCT procedure will involve a "control sample" and a "sample from the subject". The "sample from the subject" is a sample from the subject that is to be tested. For each sample, a target gene (here: the gene of interest) and a control endogenous gene (as described below) for the amplification of the PCR from aliquots (usually serial dilutions) are included. Several replicas of each diluted concentration are usually used to derive the amplification effectiveness. The PCR amplification effectiveness can be defined as the amplification percentage (from 0 to 1). During the reaction of the qPCR, a software typically measures the number of cycles of each sample in which the fluorescence crosses an arbitrary line (indicator of the PCR amplification), i.e., the threshold. This crossing point is the Ct value. More diluted samples will cross to subsequent Ct values. In order to quantify the gene expression of a particular gene, Ct of a nucleic acid from the gene of interest is divided by Ct of the nucleic acid from the endogenous control in the same sample to normalise the variation in the amount and quality of RNA among different samples and obtain the relative expression (with respect to the endogenous control) of each of the "samples from the subject" and of the "control sample". Optionally, this is conducted in duplicate, triplicate, quadruplicate and in a similar way, respectively. A ΔΔCT control value can be properly obtained by calculating the average of the ΔΔCT values obtained from samples from a group of control of several individuals with whom the values of the "sample from the subject" are going to be compared. The control group (of which the average value is calculated) consists of the appropriate individuals for the respective purposes (comparison). The person skilled in the art will learn from this disclosure that a proper control group is for a particular purpose. In a particular embodiment, the present invention can be implemented by omitting the determination of the ΔCt value of the control group, i.e., by determining (only) the ΔCt value of the "sample from the subject" and then comparing this with the respective average ΔCt value of the control shown in the examples.

As has been explained, other methods are also available in the state of the art, as for example the Northern Blot Transfer, or microarrays.

Therefore, in another preferred embodiment of this aspect of the invention, the detection of the product of GATA-4 gene expression is performed by Northern Blot Transfer. In another aspect of the invention, the detection of the product of the expression of the GATA-4 gene is performed using microarrays.

The GATA-4 gene, or GATA binding protein 4 (also known as ASD2; VSD1) codifies a member of the GATA family of zinc-finger transcription factors. Members of this family recognise the GATA motif, which is present in the promoters of many genes. This protein is believed to regulate genes involved in embryogenesis and myocardium differentiation and function. Mutations in this gene have been associated with cardiac septal defects. It is located on human chromosome 8 (8p23.1-p22).

In the context of the present invention, GATA-4 is also defined by a sequence of nucleotides or polynucleotide, which constitutes the coding sequence of the protein in the SEQ ID NO: 1, and which would include different variants from:
a) nucleic acid molecules codifying a polypeptide comprising the amino acid sequence of SEQ ID NO: 1,
b) nucleic acid molecules whose complementary chain hybrids with the polynucleotide sequence of a),
c) nucleic acid molecules whose sequence differs from a) and/or b) due to the degeneracy of the genetic code,
d) nucleic acid molecules codifying a polypeptide comprising the amino acid sequence with an identity of at least 80 %, 90 %, 95 %, 98 % or 99 % with the SEQ ID NO: 1, and in which the polypeptide encoded by said nucleic acids exhibits the activity and structural characteristics of the GATA-4 protein. Among these nucleic acid molecules, is that included in SEQ ID NO: 2.

The term "diagnosis", as used in the present invention, refers to the ability to discriminate between individuals affected or not by hepatic fibrosis or cirrhosis, and/or the ability to discriminate between the different stages of hepatic fibrosis (FI, FII, FIII or FIV). In particular, the kit of the invention allows to discriminate between the stage I (FI), stages II and III (FII and Fill), and cirrhosis (FIV).

In the present invention "prognosis" means the expected evolution of a disease and refers to the assessment of the probability according to which an individual suffers from a disease as well as to the valuation of its onset, development, evolution, or regression, and/or the prognosis of the course of the disease in the future. As will be apparent to those skilled in the art, although such valuation is preferred to be correct for 100 % of subjects to be diagnosed, it may be found that it is not. The term, however, requires that a statistically significant portion of subjects can be identified as suffering from the disease or having predisposition to it. The person skilled in the art can simply determine whether a part is statistically significant by using several well-known statistical evaluation tools, such as, for example, determination of confidence intervals, determination of p-values, Student's t test, Mann-Whitney test, etc. Preferred confidence intervals are at least 50 %, at least 60 %, at least 70 %, at least 80 %, at least 90 %, and at least 95 %. The values of p are, preferably, 0.2, 0.1, 0.05.

"Prediction of the response" means, in the context of the present invention, the determination of the likelihood that the patient will respond in a favourable or unfavourable way to a therapy or a particular treatment, including surgical treatment. Especially, the term "prediction", as used herein, refers to an individual assessment of any parameter that may be useful in determining a patient's evolution. As will be apparent to those skilled in the art, although the prediction of the clinical response to the treatment is preferred to be correct for 100 % of subjects to be diagnosed or evaluated, it is not so needed. The term, however, requires that a statistically significant part of the subjects can be identified as having an increased probability of having a positive response. The person skilled in the art can easily determine whether a subject is statistically significant using several well-known statistical evaluation tools, such as, for example, determination of intervals of confidence, determination of p values, Student's t test, Mann-Whitney test, etc. Preferred confidence intervals are at least 50 % >, at least 60 % >, at least 70 % >, at least 80 % >, at least 90 %>, at least 95 % >. The values of p are, preferably, 0.2, 0.1, or 0.05. The prediction of the clinical response can be done by using any endpoint used in hepatology and known to the person skilled in the art.

Also, according to the method of the present invention, other sub-classifications within this main one could be established, thus facilitating the election and the establishment of appropriate therapeutic regimens or treatment. This discrimination, as it is understood by a person skilled in the art, is not intended to be 100 % correct for the samples analysed. However, it requires that a statistically significant number of analysed samples are correctly classified. The amount that is statistically significant can be established by a person skilled in the art through the use of various statistical tools, for example, but without limitation, through the determination of confidence intervals, determination of the significance value P, Student's test or Fisher's discriminant functions, Mann Whitney non-parametric measures, Spearman's correlation, logistic regression, linear regression, area under a ROC curve (AUC). Preferably, the confidence intervals are at least 90 %, at least 95 %, at least 97 %, at least 98 % or at least 99 %. Preferably, the p value is less than 0.1, than 0.05, than 0.01, than 0.005 or than 0.0001. Preferably, the present invention allows to correctly detecting the disease in a differential way in at least 60 %, most preferably in at least 70 %, much more preferably in at least 80 %, or even more preferably in at least 90 % of the subjects of a particular group or population analysed.

The term "individual", as used in the description, refers to animals, preferably mammals, and more preferably, humans. The term "individual" is not intended to be limiting in any aspect, and can be of any age, sex and physical condition.

The amount of reference is obtained from the values of constitutive expression of genes, in a group of healthy individuals, or the expression of genes in the group of individuals before being subjected to the treatment.

The amount of reference will be, for example, in the case of the differentiation between patients affected by hepatic fibrosis or cirrhosis and healthy individuals, the constitutive expression of the gene or the amount of protein detected in a control group of healthy individuals. However, in the case of the sub-classification of patients affected by hepatic fibrosis in different stages, the control group will be formed by a group of patients with hepatic fibrosis at a certain development stage. In another preferred embodiment of this aspect of the present invention, the amount of reference is obtained from a reference sample. The amount of reference can be obtained, for example, from the limits of normal distribution of an amount found in samples obtained from a population of individuals with hepatic fibrosis, or hepatic fibrosis in different stages, using well-known statistical techniques. In another preferred embodiment, the reference sample is obtained from patients before and after treatment.

Another aspect of the invention relates to the use of a pharmaceutical composition comprising a modulating agent for the GATA-4 gene in the preparation of a drug to treat an individual diagnosed of hepatic fibrosis or cirrhosis using the methods of the invention, or alternatively, to a pharmaceutical composition comprising a modulating agent for the GATA-4 gene, to treat an individual diagnosed of hepatic fibrosis or cirrhosis using the methods of the invention. Preferably, the modulating agent is an inhibitor of the expression of said gene.

### Quantificafion of the protein

The quantification of the amount of GATA-4 protein can be made by any of the techniques known to the person skilled in the art, preferably by immunological techniques.

In a more preferred embodiment, the immunological techniques are based on precipitation reactions, agglutination reactions, immunolabeling, radioimmunoassay, and radioimmunometric techniques, ELISA (Enzime Linked ImmunoadSorbent Assay), or any combination thereof. In another more preferred embodiment, the immunological techniques comprise immunolabeling. In a still more preferred embodiment, the immunolabeling is selected among immunolabeling with antibodies conjugated to enzymes, immunolabeling with antibodies conjugates to fluorochromes, or cytometry. Even more preferably, cytometry is flow cytometry.

### DIAGNOSTIC KIT OR DEVICE, MICROARRAY OR PROTEIN MICROARRAY AND USES THEREOF

Another aspect of the present invention relates to a kit or device, from now on kit or device of the invention, which comprises the elements needed to analyse the expression level of the GATA-4 gene. In another preferred embodiment, the kit may contain oligonucleotides designed from a known sequence or a mRNA gene, and/or able to hybridise with the sequence of the GATA-4 gene, for subsequent amplification by PCR. More preferably, the sequence of the GATA-4 gene is the nucleotide sequence SEQ ID NO: 2.

In another preferred embodiment, the kit or device of the invention includes at least one anti-GATA-4 antibody. In a preferred embodiment of this aspect of the invention, the antibody is human, humanised or synthetic. In another more preferred embodiment, the antibody is monoclonal. In another more preferred embodiment, the antibody is labelled with a fluorochrome. More preferably, the fluorochrome is selected from the list comprising Fluorescein (FITC), Tetramethylrhodamine and derivatives, Phycoerythrin (PE), PerCP, Cy5, Texas, allophycocyanin, or any combination thereof.

More preferably, it comprises the means required to compare the amount detected in step (b) with a reference amount.

This kit may contain all the reagents necessary to analyse the expression level of the GATA-4 gene, by any of the methods described herein above. The kit may also include, without any limitation, buffers, agents to prevent contamination, inhibitors of protein degradation, etc. Also, the kit may include all the carriers and containers necessary for its implementation and optimisation. Preferably, the kit comprises also the instructions to carry out any of the methods of the invention.

In the case of (a) a kit suitable for the RQ-PCR, which is a sensitive and reproducible quantification technique of gene expression, it is desired that the kit further comprises a poly-T oligonucleotide primer besides the oligonucleotide(s) of the kit. These reagents can be optionally comprised in the kit.

A Northern Transfer involves the use of electrophoresis to separate RNA samples by size and the subsequent detection with one or more oligonucleotides (hybridisation probe) complementary with (part of) the target sequence of the RNA of interest.

It is also possible that the oligonucleotide(s) are immobilised in spots on a (preferably solid) surface. In one of its embodiments, the kit includes a microarray, or microarray of the invention. A microarray of RNA is a matrix on a solid substrate (usually a glass slide or a cell in a thin film of silicon) that assesses large amounts of different RNA that are detectable by means of specific probes immobilised on spots on a solid substrate. Each spot contains a specific nucleic acid sequence, typically a DNA sequence, as probes (or indicators). Although the number of spots is not limited in any way, in a preferred embodiment, the microarray is customised for the invention methods. In one embodiment, this customised array comprises fifty spots or less, such as thirty spots or less, including twenty spots or less. Therefore, another aspect of the invention relates to a microarray comprising oligonucleotides designed from a known sequence or a mRNA of the genes, and/or able to hybridise with the sequence of the GATA-4 gene. More preferably, the sequence of the GATA-4 gene is the nucleotide sequence SEQ ID NO: 2.

Another aspect of the invention relates to a microarray, hereinafter microarray of the invention, comprising oligonucleotides or single-channel microarrays designed from a known sequence or a mRNA of the GATA-4 gene. More preferably, the sequence of the GATA-4 gene is the nucleotide sequence SEQ ID NO: 2.

Thus, for example, oligonucleotides sequences are built on the surface of a chip using the sequential elongation of a growing chain with a single nucleotide using photolithography. Thus, the oligonucleotides are anchored by the 3' end by means of a method of selective activation of nucleotides, protected by a photolabile reagent, through the selective incidence of light through a photomask. The photomask can be physical or virtual.

Thus, oligonucleotide probes can be between 10 and 100 nucleotides, most preferably between 20 and 70 nucleotides, and even more preferably, between 24 and 30 nucleotides. For the quantification of gene expression, preferably about 40 oligonucleotides are used per gene.

Synthesis in situ on a solid base (e.g., glass), could be done by ink-jet technology, which requires longer probes. Carriers may be, without limitation, filters or membranes of NC or nylon (loaded), silicon, or glass slides for microscopes covered with aminosilanes, polylysine, aldehydes, or epoxy. The probe is each one of the samples of the chip. The target is the sample to be analysed: messenger RNA, total RNA, a fragment of PCR, etc.

Another aspect of the invention relates to a protein microarray, hereinafter protein microarray of the invention, which comprises anti-GATA-4 antibodies. The probes are antibodies attached to the glass slide and blanks are serum or tissue samples.

Another aspect of the invention relates to the use of the kit or device, the microarray, or microarray of invention, for obtaining useful data in the diagnosis of hepatic fibrosis or cirrhosis.

Another aspect of the invention relates to a storage medium readable by a computer comprising software instructions capable of making a computer carry out the steps of any of the methods of the invention (the first or the second method of the invention).

Another aspect of the invention relates to a transmissible signal comprising software instructions capable of making a computer carry out the steps of any of the methods of the invention.

The terms "polynucleotide" and "nucleic acid" are interchangeably used herein to refer to polymeric forms of nucleotides of any length, both ribonucleotides (RNA or RNA) and deoxyribonucleotides (DNA or DNA). The terms "amino acid sequence", "peptide", "oligopeptide", "polypeptide" and "protein" are interchangeably used herein and refer to a polymer form of amino acids of any length, which can be coding or non-coding, chemical or biochemically modified.

Throughout the description and claims the word "comprise" and its variations are not intended to exclude other technical characteristics, additives, components or steps. For those skilled in the art, other objects, advantages and features of the invention will partly arise from the description and partly from the practice of the invention. The following examples and drawings are provided as an illustration, and are not intended to be limiting of the present invention.

### BRIEF DESCRIPTION OF THE DRAWINGS

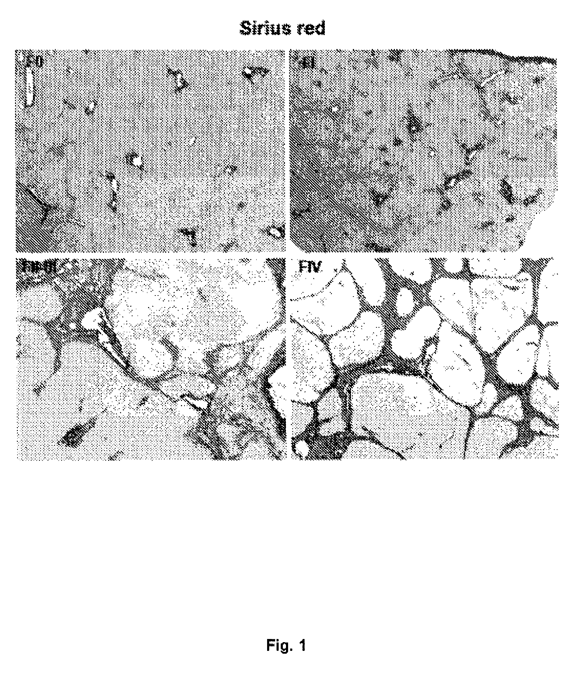

**Fig. 1****. Histological analysis of tissue sections in paraffin stained with Sirius red from biopsies of human liver from healthy patients (normal), with fibrosis type I (FI), fibrosis type II-III (FII-III) and cirrhosis.** Collagen fibres dyed with Sirius red reflecting the degree of fibrosis and cirrhosis are displayed in dark gray/black.

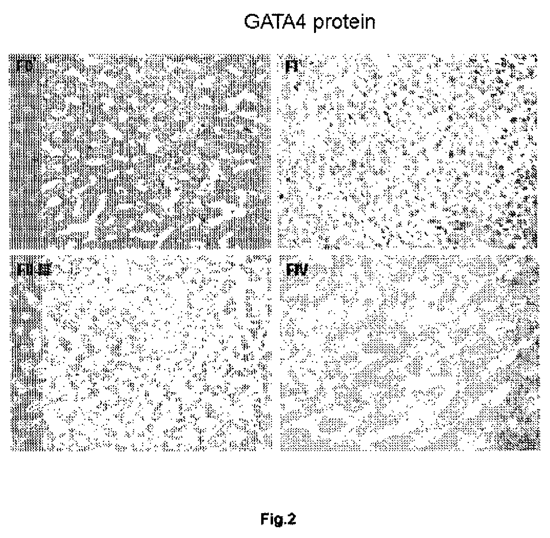

**Fig. 2****. Immunohistochemical analysis of tissue sections in paraffin from biopsies of human liver from healthy patients (normal), with fibrosis type I (FI), fibrosis type II-III (FII-III) and cirrhosis.** The immunodetection of GATA-4 in the nucleus of the stellate cells is observed as a black dot in the sinusoidal spaces between the hepatocytes.

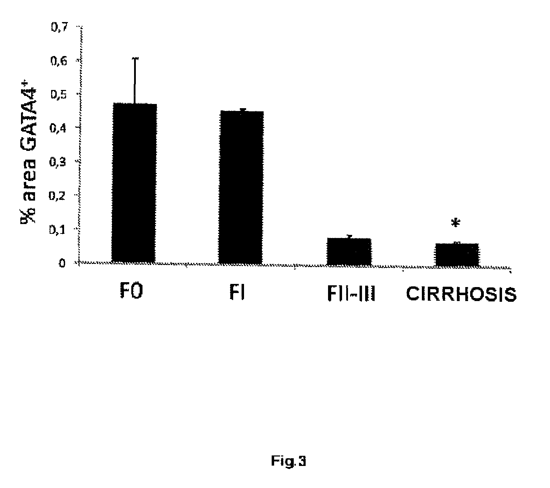

**Fig. 3****. Quantitative analysis of positive cells for GATA4 in biopsies of human liver from healthy patients (FO), with fibrosis type I (FI), fibrosis type II-III (FII-III) and cirrhosis.**

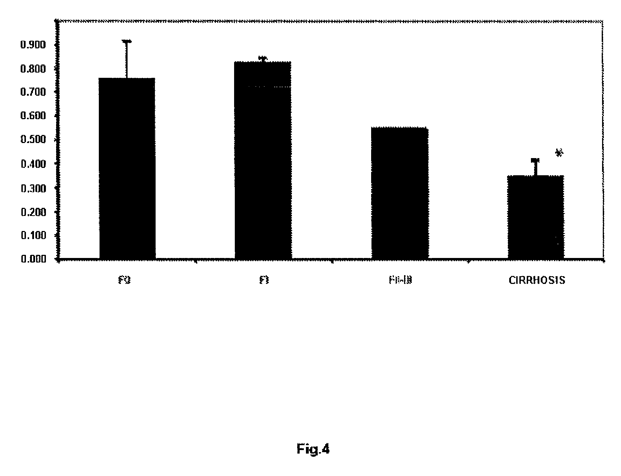

**Fig. 4****. Quantitative analysis of levels of messenger RNA of the GATA-4 gene in liver tissue from biopsies of human liver from healthy patients (normal), with fibrosis type I (FI), fibrosis type II-III (FII-III) and cirrhosis.**

### EXAMPLES OF THE INVENTION

Now, the invention will be illustrated by tests carried out by the inventors, which evidence the specificity and effectiveness of the methods of the invention to obtain data useful in the differential diagnosis of hepatic fibrosis.

### Methodology

### Collection and preparation of samples from human liver

The human liver samples were obtained by wedge biopsies or transplant hepatotectomy. Samples were collected from:
- 10 healthy patients by routine biopsy
- 5 samples from patients with hepatic fibrosis of undetermined etiology. The fibrosis were classified according to the METAVIR system as:
   Liver in fibrosis stage (1 sample)
   Portal fibrosis of mild or residual type (4 samples)
   Pre-cirrhotic portal fibrosis, stage III (6 samples)
- 7 samples of hepatic cirrhosis, 3 of them of alcoholic etiology and 2 of them of viral etiology caused by the virus of hepatitis C.

All samples were fixed in 10 % neutral formalin for 36 hours approximately. Fixed samples were embedded in paraffin and cut in 5 µm-thick sections for subsequent histological and immunohistochemical analysis. Hepatic tissue sections were mounted on slides previously xylanised with 2 % 3-amino-propyl-triethoxy-xylan (APES) (Sigma Aldrich catalogue number A3648) and incubated at 60°C for 12 hours.

### Histological analysis.

Samples were deparaffinised through several washing in xylene and ethanol at different concentrations and then dyed according to the following protocol:
1. Immersing samples in xylene: 10 minutes
2. Immersing samples in 100 % ethanol: 5 minutes
3. Immersing samples in 90 % ethanol: 5 minutes
4. Immersing samples in 70 % ethanol: 5 minutes
5. Washing samples with distilled water: 10 minutes.
6. Immersing samples in Weigert's iron hematoxylin (HT1079, Sigma Aldrich) for 8 minutes.
7. Performing continuous washing of the samples with tap water (not distilled water) for 10 minutes.
8. Immersing samples in picro-sirius red solution for one hour. The solution picro- sirius red is prepared as follows: 0.5 g Sirius red (Direct Red 80, number 365548, Sigma Aldrich catalogue) in 500 ml of picric acid (catalogue number P6744, Sigma Aldrich).
9. Performing two washes of 5 seconds each in acidic water (acidic water is prepared by adding 5 ml of acetic acid in 995 ml of distilled water).
10. Dehydrating tissue by passing it 5 minutes in 70 % ethanol, 5 minutes in 90 % ethanol and 5 minutes in 100 % ethanol.
11. Immersing the samples in xylene for 5 minutes.
12. Mounting the samples by adding mounting DPX medium (catalogue number HT1079, Prolabo) and cover with a coverslip.

The photographs were taken on a Leica AF6000 microscope with DFC500 camera using polarised light and 10x lenses.

### Immunohistochemical analysis

The deparaffined samples obtained in step 5 of the previous protocol were subject to immunohistochemical analysis according to the following protocol:
1. The samples were immersed in a solution of 10mM citrate at pH 6 and heated in an autoclave at 121°C for 40 minutes to unmask the epitopes. (10mM sodium citrate, S-4611, Sigma Aldrich.) To reach pH 6 a solution of citric acid, C-0759, Sigma Aldrich is used).
2. After heating, the samples were left to cool at room temperature, washed twice with distilled water and incubated in 3 % H₂O₂ diluted in distilled water for 10 minutes to block endogenous peroxidase activity.
3. Samples were then blocked in 3 % Donkey Serum (catalogue number D9663, Sigmal Aldrich) diluted in Phosphate Buffered Saline solution (PBS, catalogue number P5368, Sigma Aldrich) and adding 0.2 % Triton X-100 (catalogue number A4975, AppliChem) for 1 hour at room temperature.
4. After blocking incubation, the samples were incubated with monoclonal mouse anti-GATA-4 antibody (G-4, Sc-25310, Santa Cruz Biotechnologies) using a dilution 1:50 at 4°C overnight. The antibody was diluted in PBS+3 % NGS+0.2 % Triton X-100.
5. After incubation with antibody, the samples were washed three times for 5 minutes with PBS and incubated with secondary anti-mouse biotinylated antibody (catalogue number BA-2020, Vector Laboratories, Inc.) to a dilution 1:300 in PBS+3 % NGS+0.2 % Triton X-100 at room temperature for 1 hour.
6. Then, the samples were washed with PBS three times for 5 minutes each washing and finally washed in distilled water and incubated with Vectastain Elite ABC immunoperoxidase (catalogue number PK-6100, Vector Laboratories), following the manufacturer's instructions.
7. The samples were washed three times with PBS and were revealed using diaminobenzidine (DAB) according to the instructions of the Peroxidase Substrate DAB kit (catalogue number sk-4100, Vector Laboratories, Inc.)
8. The samples were dehydrated by passing them for 5 minutes in 90 °/fl ethanol, 70 % ethanol, 100 % ethanol and xylene and finally mounted with DPX in order to photograph them in the Leica AF6000 microscope with DFC500 camera using 10x, 20x and 40x lenses.

### Quanfifative analysis of positive cells for GATA4 in biopsies previously analysed using immunohistochemical techniques

Photographs taken in the previous section of all liver biopsies tested by technical Immunohistochemistry for detecting GATA4, were processed using the software MetaMorph 7.1. Positive pixels for diaminobenzidine (DAB), which label the positive cells for GATA4, were selectively detected using the threshold function of the software MetaMorph 7.1. The positive area for GATA4 was obtained as the ratio of positive pixels for DAB and the number of pixels in the complete image. The asterisk indicates statistical significance between the levels of expression of GATA-4 in normal patients and cirrhotic patients with a probability of p = 0.05 in a Student's Test for non-paired samples.

### Quantitafive analysis of messenger RNA

The total RNA of samples from biopsies of livers from healthy patients (FO), with fibrosis type I, fibrosis type II-III or cirrhosis was obtained using the mirVana miRNA islolation kit (Ambion, catalogue number 1560). In all cases, 10-13 sections of 20 microns thick of fresh liver samples were used. Biopsies were collected form 5 healthy patients (F0), 2 patients with fibrosis type I, one patient with fibrosis type II-III and 5 patients with cirrhosis. Retrotranscription reactions were conducted using QUANTITEC REVERSE TRANSCRIPTION kit (Qiagen, catalogue number 205313). For the polymerase chain reaction, Taqman gene expression master mix (Applied Biosystem, catalogue number 436016) and commercial Taqman probes were used for the amplification of the GATA-4 gene (Gata-4 Hs00171403_m1 Applied Biosystem) and for the amplification of B-actin (Hs03023880_g1 Applied Biosystem) internal reference gene. The polymerase chain reaction was carried out in a thermal cycler Real Time PCR 700 from Applied Biosystem. The asterisk indicates statistical significance between the levels of expression of GATA-4 in normal patients and cirrhotic patients with a probability of p = 0.05 in a Student's Test for non-paired samples. The quantification of the expression levels of GATA-4 have been made in triplicate for each sample and in three independent experiments.

pctes2014070978 seql - English translation
SEQUENCE LISTING

<110>  Fundación Pública Andaluza Progreso y Salud
<110>  Fundación Pública Andaluza para la Gestión de la Investigación en Salud
de Sevilla
<110>  Universidad Pablo de Olavide

<120>  METHOD FOR OBTAINING USEFUL DATA FOR THE DIFFERENTIAL
       DIAGNOSIS OF LIVER FIBROSIS

<130>  PCT-06836

<160>  2

<170>  PatentIn version 3.5

<210>  1
<211>  442
<212>  PRT
<213>  Homo sapiens

<400>  1

<210>  2
<211>  3419
<212>  DNA

<213>  Homo sapiens

<400>  2
ttggaggcgg ccggcgcagg ggccgcgaga ggcttcgtcg ccgctgcagc tccgggggct      60

cccaggggag cgtgcgcgga acctccaggc ccagcaggac cccggctgcg gcgaggagga     120

aggagccagc ctagcagctt ctgcgcctgt ggccgcgggt gtcctggagg cctctcggtg     180

tgacgagtgg gggacccgaa ggctcgtgcg ccacctccag gcctggacgc tgccctccgt     240

cttctgcccc caataggtgc gccggacctt caggccctgg ggtgaattca gctgctccta     300

catcagcttc cggaaccacc aaaaattcaa attgggattt tccggagtaa acaagagcct     360

agagcccttt gctcaatgct ggatttaata cgtatatatt tttaagcgag ttggtttttt     420

cccctttgat tttgatcttt cgcgacagtt cctcccacgc atattatcgt tgttgccgtc     480

gttttctctc cccgcgtggc tccttgacct gcgagggaga gagaggacac cgaagccggg     540

agctcgcagg gaccatgtat cagagcttgg ccatggccgc caaccacggg ccgccccccg     600

gtgcctacga ggcgggcggc cccggcgcct tcatgcacgg cgcgggcgcc gcgtcctcgc     660

cagtctacgt gcccacaccg cgggtgccct cctccgtgct gggcctgtcc tacctccagg     720

gcggaggcgc gggctctgcg tccggaggcg cctcgggcgg cagctccggt ggggccgcgt     780

ctggtgcggg gcccgggacc cagcagggca gcccgggatg gagccaggcg ggagccgacg     840

gagccgctta caccccgccg ccggtgtcgc cgcgcttctc cttcccgggg accaccgggt     900

ccctggcggc cgccgccgcc gctgccgcgg cccgggaagc tgcggcctac agcagtggcg     960

gcggagcggc gggtgcgggc ctggcgggcc gcgagcagta cgggcgcgcc ggcttcgcgg    1020

gctcctactc cagcccctac ccggcttaca tggccgacgt gggcgcgtcc tgggccgcag    1080

ccgccgccgc ctccgccggc cccttcgaca gcccggtcct gcacagcctg cccggccggg    1140

ccaacccggc cgcccgacac cccaatctcg atatgtttga cgacttctca gaaggcagag    1200

agtgtgtcaa ctgtggggct atgtccaccc cgctctggag gcgagatggg acgggtcact    1260

atctgtgcaa cgcctgcggc ctctaccaca agatgaacgg catcaaccgg ccgctcatca    1320

agcctcagcg ccggctgtcc gcctcccgcc gagtgggcct ctcctgtgcc aactgccaga    1380

ccaccaccac cacgctgtgg cgccgcaatg cggagggcga gcctgtgtgc aatgcctgcg    1440

gcctctacat gaagctccac ggggtcccca ggcctcttgc aatgcggaaa gaggggatcc    1500

aaaccagaaa acggaagccc aagaacctga ataaatctaa gacaccagca gctccttcag    1560

gcagtgagag ccttcctccc gccagcggtg cttccagcaa ctccagcaac gccaccacca    1620

gcagcagcga ggagatgcgt cccatcaaga cggagcctgg cctgtcatct cactacgggc    1680

acagcagctc cgtgtcccag acgttctcag tcagtgcgat gtctggccat gggccctcca    1740

tccaccctgt cctctcggcc ctgaagctct ccccacaagg ctatgcgtct cccgtcagcc    1800

agtctccaca gaccagctcc aagcaggact cttggaacag cctggtcttg gccgacagtc    1860

acggggacat aatcactgcg taatcttccc tcttccctcc tcaaattcct gcacggacct    1920

gggacttgga ggatagcaaa gaaggaggcc ctgggctccc aggggccggc ctcctctgcc    1980

pctcs2014070378 seql - English translation
tggtaatgac tccagaacaa caactgggaa gaaacttgaa gtcgacaatc tggttagggg 2040
aagcgggtgt tggattttct cagatgcctt tacacgctga tgggactgga gggagcccac 2100
ccttcagcac gagcacactg catctctcct gtgagttgga gacttctttc ccaagatgtc 2160
cttgtcccct gcgttcccca ctgtggccta gaccgtgggt tttgcattgt gtttctagca 2220
ccgaggatct gagaacaagc ggagggccgg gccctgggac ccctgctcca gcccgaatga 2280
cggcatctgt ttgccatgta cctggatgcg acgggcccct ggggacaggc ccttgcccca 2340
tccatccgct tgaggcatgg caccgccctg catccctaat accaaatctg actccaaaat 2400
tgtggggtgt gacatacaag tgactgaaca cttcctgggg agctacaggg gcacttaacc 2460
caccacagca cagcctcatc aaaatgcagc tggcaacttc tcccccaggt gccttccccc 2520
tgctgccggc ctttgctcct tcacttccaa catctctcaa aataaaaatc cctcttcccg 2580
ctctgagcga ttcagctctg cccgcagctt gtacatgtct ctcccctggc aaaacaagag 2640
ctgggtagtt tagccaaacg gcaccccctc gagttcactg cagacccttc gttcaccgtg 2700
tcacacatag aggggttctg agtaagaaca aaacgttctg ctgctcaagc cagtctggca 2760
agcactcagc ccagcctcga ggtccttctg gggagagtgt aagtggacag agtcctggtc 2820
agggggcagg agtgtcccaa gggctggccc acctgctgtc tgtctgctcc tcctagccct 2880
tggtcagatg gcagccagag tccctcagga cctgcagcct cgccccggca gaagtctttt 2940
gtccaggagg caaaaagcca gagattctgc aacacgaatt cgaagcaaac aaacacaaca 3000
caacagaatt cctggaaaga agacgactgc taagacacgg caggggggcc tggagggagc 3060
ctccgactct gagctgctcc gggatctgcc gcgttctcct ctgcacattg ctgtttctgc 3120
ccctgatgct ggagctcaag gagactcctt cctctttctc agcagagctg tagctgactg 3180
tggcattact acgcctcccc acacgcccag acccctcact ccaaaatcct actggctgta 3240
gcagagaata cctttgaacc aagattctgt tttaatcatc atttacattg ttttcttcca 3300
aaggccccct cgtataccct ccctaaccca caaacctgtt aacattgtct taaggtgaaa 3360
tggctggaaa atcagtattt aactaataaa tttatctgta ttcctcttaa aaaaaaaaa 3419

## Claims

1. Use of the GATA-4 gene, or GATA-4 protein for the differential diagnosis of hepatic fibrosis.

2. A method for obtaining useful data for the differential diagnosis of hepatic fibrosis comprising:
a. obtaining an isolated biological sample comprising cells from an individual, and
b. detecting the level of expression of the GATA-4 gene, and/or the amount of GATA-4 protein, in the isolated sample of (a), and
c. comparing the gene expression obtained in step (b) with a reference amount.

3. A method for the differential diagnosis of hepatic fibrosis, which comprises the steps (a) - (c) according to claim 2, and further comprises:
d) diagnosing the individual of step (a) as an individual with hepatic fibrosis type I, when it presents an increased expression of the GATA-4 gene or a larger amount of GATA-4 protein in the sample obtained in (a), in relation to the amount of expression detected for said gene or protein in a population of patients (F0) of reference.

4. The method according to Claim 3, wherein the individual of step (a) presents an expression level of the GATA-4 gene of 110 % over the expression levels in a population of patients (F0) of reference.

5. The method according to any of claims 3-4, further comprising diagnosing the individual of step (a) as an individual with portal fibrosis with numerous septae without cirrhosis (FII-FIII), when it presents an expression level of the GATA-4 gene between 15 % and 25 % less than in an individual of reference.

6. The method according to claim 5, wherein the individual of step (a) presents an expression level of the GATA-4 gene about 20 % less than in an individual of reference.

7. The method according to any of claims 3-6, further comprising diagnosing the individual of step (a) as an individual with cirrhosis (FIV), when it presents an expression level of the GATA-4 gene between 55 % and 65 % less than an individual of reference.

8. The method according to claim 7, wherein the individual of step (a) presents an expression level of the GATA-4 gene about 60 % less than in an individual of reference.

9. The method according to any of claims 3-8, wherein the isolated biological sample from an individual of step (a) is a sample of liver tissue.

10. The method according to any of claims 3-9, wherein the detection of the expression levels of the GATA-4 gene is performed using Q-RT-PCR.

11. The method according to any of claims 3-10, wherein the identification of the amount of the GATA-4 protein is performed using immunological techniques.

12. The method according to claim 11, wherein the immunological techniques are based on precipitation reactions, agglutination reactions, immunolabeling, radioimmunoassay, and radioimmunometric techniques, ELISA (Enzime Linked ImmunoadSorbent Assay), or any combination thereof.

13. The method according to any of claims 11-12, wherein the immunological techniques comprise immunolabeling.

14. The method according to claim 13, wherein the immunolabeling is selected among immunolabeling with antibodies conjugated to enzymes, immunolabeling with antibodies conjugates to fluorochromes, or cytometry.

15. A kit or device comprising the elements needed to analyse the expression level of the GATA-4 gene or the amount of GATA-4 protein.

16. The kit or device according to the previous claim, comprising an anti-GATA-4 antibody.

17. The kit or device according to any of claims 15-16, wherein the antibody is monoclonal.

18. The kit or device according to any of claims 15-17, wherein the antibody is labelled with a fluorochrome.

19. The kit or device according to the previous claim, wherein the fluorochrome is selected from the list comprising Fluorescein (FITC), Tetramethylrhodamine and derivatives, Phycoerythrin (PE), PerCP, Cy5, Texas, allophycocyanin, or any combination thereof.

20. The use of the kit or device according to any of claims 15-19 for the differential diagnosis of hepatic fibrosis, mainly for fibrosis type I, fibrosis type II-III, and cirrhosis.
